# EUROPEAN PATENT APPLICATION

(11) **EP 1 279 728 A1**
(43) Date of publication of application: **29.01.2003**
(21) Application number: 01118199.7
(22) Date of filing: 27.07.2001
(51) Int. Cl.: C12N 5/06, C12N 5/08, C12M 3/04, C12M 1/14, C12M 1/20, C12M 1/24, A61K 35/14

(54) **Generation of fully mature and stable dentritic cells from leukapheresis products for clinical applications**

(71) Applicant: Schuler, Gerold, 91080 Spardorf (DE)
(72) Inventor: Schuler, Gerold Prof. Dr., 91080 Spardorf (DE); Thurner-Schuler, Beatrice Dr., 91080 Spardorf (DE); Berger, Thomas Dr., 91054 Erlangen (DE)
(74) Representative: Helbing, Jörg, Dr. Dipl.-Chem.

(57) **Abstract**

The present invention provides a method for producing mature and stable dendritic cells or immature dendritic cells which comprises cultivating hematopoietic progenitor cells in a sterile cultivating apparatus, an apparatus suitable for said method and a method for preparing peripheral blood mononuclear cells, which are suitable for cultivation of dendritic cells.

## Description

The present invention provides a method for producing mature and stable dendritic cells or immature dendritic cells which comprises cultivating hematopoietic progenitor cells in a sterile cultivating apparatus, an apparatus suitable for said method and a method for preparing peripheral blood mononuclear cells, which are suitable for cultivation of dendritic cells.

### Discussion of the Related Art

Dendritic cells (DC) constitute a system of antigen-presenting cells that control immunity by interacting with lymphocytes. Most DC are myeloid-derived and immunostimulatory (Banchereau, J., Steinman, R.M., Nature, 392, 245 (1998)). These classical DC are specialized in several ways to prime helper and killer T cells *in vivo* ("nature's adjuvant"). Most importantly, immature DC that reside in peripheral tissues are equipped to capture antigens and to produce immunogenic MHC-peptide complexes ("antigen-processing mode"). In response to maturation-inducing stimuli such as inflammatory cytokines ("danger") these immature DC develop into potent T cell stimulators by upregulating adhesion and costimulatory molecules ("T cell stimulatory mode"), and at the same time migrate into secondary lymphoid organs to select and stimulate rare antigenspecific T cells. DC that were laboriously isolated from tissue or blood, if charged with antigens *in vitro,* and injected back as mature DC proved immunogenic (Inaba, K. et al., J. Exp. Med., 178, 479 (1993); Inaba, K. et al., Int. Rev. Immunol., 6, 197 (1990); Hsu, F.J. et al., Nature Med., 2, 52 (1996)) *in vivo.* These data suggest that DC are effective adjuvants for immune-mediated resistance to tumors and infections.

The development of methods to generate DC *ex vivo* in large numbers from hematopoietic progenitors was a prerequiste to explore such DC-based vaccination approaches in more detail. Following the discovery that GM-CSF was the key cytokine for DC-generation from murine blood (Inaba, K. et al., J. Exp. Med., 175, 1157 (1992)) a simple and reliable technique to generate (primarily mature) DC from murine bone marrow (Inaba, K. et al., J. Exp. Med., 176, 1693 (1992)) was developed. Several groups then uniformly demonstrated that the injection of such DC progeny if charged with tumor antigen induced CD8+ CTL-mediated tumor regression in mice (reviewed in Young, J. W., Inaba, K., J. Exp. Med., 183, 7 (1996); Schuler, G., Steinman, R.M., J. Exp. Med., 186, 1183 (1997)). Methods to generate human DC have also been worked out but are not yet as standardized as in the mouse. DC generated ex vivo from precursors have already been used successfully to vaccinate humans and to treat disease (Bancherau, J. et al., Cell, in press, 2001).

In man DC can be generated either from rare, proliferating CD34+ precursors by using GM-CSF + TNF alpha as key cytokines (Caux, C. et al., Nature, 360, 258 (1992); Siena, S. et al., Exp. Hematol., 23, 1463 (1995); WO 93/20185) or from more frequent, but non-proliferating CD14+ precursors (monocytes) in peripheral blood under the aegis of GM-CSF + IL-4 (WO 97/29182). The latter method has been used widely for experimental purposes since its introduction in 1994 (Sallusto, F., Lanzavecchia, A., J. Exp. Med., 179, 1109 (1994); Romani, N. et al., J. Exp. Med., 180, 83 (1994)), and for a number of reasons appears also attractive for immunotherapy. First, the CD14+ precursors are abundant so that pretreatment of patients with cytokines such as G-CSF (used to increase CD34+ cells and more committed precursors in peripheral blood) is unnecessary in most cases (Romani, N. et al., J. Immunol. Methods, 196, 137 (1996)). Secondly, the DC generated by this approach appear rather homogenous and can be produced in an immature state or fully differentiated or mature. Thirdly, it was shown that it is possible to avoid non-human proteins such as FCS (fetal calf serum), and to obtain fully and irreversibly mature and stable DC by using autologous monocyte conditioned medium as maturation stimulus (Romani, N. et al., J. Immunol. Methods, 196, 137 (1996); Bender, A. et al., J. Immunol. Methods, 196, 121 (1996)). It is desirable to avoid FCS as it is potentially harmful (due to infectivity and immunogenicity) and non-standardized (the quality of DC progeny varies markedly depending on the particular FCS batch used).

Moreover, the following patents/patent applications disclose the production of dendritic cells:
EP-A-0 922 758 discloses the production of mature dendritic cells from immature dendritic cells derived from pluripotential cells having the potential of expressing either macrophage or dendritic cell characteristics, said method comprising contacting the immature dendritic cells with a dendritic cell maturation factor comprising IFNα.
EP-B-0 633930 discloses the production of human dendritic cells comprising the steps of
   (a) culturing human CD34⁺ hematopoietic cells (i) with GM-CSF, (ii) with TNF-α and IL-3, or (iii) with GM-CSF and TNF-α, thereby inducing the formation of CD1a⁺ hematopoietic cells; and
   (b) recovering said CD1a⁺ human dendritic cells from said culture.
WO 95/28479 discloses a process for preparing dendritic cells comprising isolation of peripheral blood cells, enriching therefrom blood precursor cells that express the CD 34 antigen and expanding said cells with a combination of hematopoietic growth factors and cytokines.

Mature DC appear preferable to immature ones for immunotherapy for a number of reasons. Only mature DC progeny lack M-CSF-R and remain stable upon removal / in the absence of M-CSF (Romani, N. et al., J. Immunol. Methods, 196, 137 (1996)). Mature DC but not immature ones are resistant to (tumor-derived) inhibitory factors such as IL-10 (Steinbrink, K. et al., J. Immunol., 159, 4772 (1997); Steinbrink, K. et al., (1998)) or VEGF (Gabrilovich et al., Nature Med., 2, 1096 (1996)). Mature DC have also been shown to be superior in inducing T cell responses *in vitro* (Sallusto, F., Lanzavecchia, A., J. Exp. Med., 179, 1109 (1994); Romani, N. et al., J. Immunol. Methods, 196, 137 (1996); Bender, A. et al., Immunol. Methods, 196, 121 (1996); Reddy, A. et al., Blood, 90, 3640 (1997); Inaba et al., J. Exp. Med., 175, 1157 (1992); Inaba et al., J. Exp. Med., 176, 1693 (1992); Larsson, M. et al., J. Immunol., 165(3):1182-90 (Aug 1, 2000); Jonuleit, H. et al., J. Exp. Med., 192(9):1213-22 (Nov 6, 2000)) and *in vivo* (.Dhodapkar, M.V. et al., J. Exp. Med., 193(2):233-8 (Jan 15, 2001) and Jonuleit, H. et al., Int. J. Cancer., 93(2):243-51 (Jul 15, 2001)). As a matter of fact, immature DC can even induce tolerance *in vitro* (Jonuleit, H. et al., J. Exp. Med., 192(9):1213-22 (Nov 6, 2000)) as well as *in vivo* (Dhodapkar, M.V. et al., J. Exp. Med., 193(2):233-8 (Jan 15, 2001)) by inducing regulatory T cells.

Mature DC are thus preferable for inducing immunity, immature DC for inducing tolerance.

The above methods for generating DC from CD14+ monocyte precursors were further modified to make it clinically practical for performing larger DC-based vaccination trials (Thurner, B. et al, J. Immunological Methods 223, 1-15 (1999)). Modifications were introduced that allow the reproducible generation of fully mature DC from leukapheresis products (rather than repeatedly drawn fresh blood as a starting population) in order to provide a reproducible DC generation method (which is essential for DC-based vaccination approaches in man) that can be performed in conformity with GMP (Good Manufacturing Practice) guidelines and that circumvents the need for multiple blood drawings to generate DC. In particular, in said method mature DC were generated from CD14+ monocytes by a two step method (priming in GM-SF + IL-4 followed by maturation in monocyte conditioned medium) for use with leukapheresis products as a starting population. Several adaptions were necessary. It was disclosed that a modified adherence step is necessary to reliably enrich CD14+ DC precursors from apheresis mononuclear cells. The addition of GM-CSF + IL-4 at the onset of culture proved disadvantageous and was, therefore, delayed for 24 hours. DC development from apheresis cells occurred faster than from fresh blood or buffy coat, and was complete after 7 days. Monocyte conditioned medium when added on day 6 resulted in fully mature and stable DC (veiled, highly migratory and T cell sensitizing cells with a characteristic phenotype such as ≥ 85% CD83+, p55 /fascin +, CD115 / M-CSF-R -, CD86++) already after 24 hours. The mature DC progeny were shown to remain stable and viable if cultured for another 1-2 days in the absence of cytokines, and to be resistant to inhibitory effects of IL-10. Freezing conditions were established to generate DC from frozen aliquots of PBMC (peripheral blood mononuclear cell(s)) or to freeze mature DC themselves for later use. The approach yields large numbers of standardized DC (5 - 10 x 10⁸ mature CD83+ DC / leukapheresis) that are suitable for performing sound Debased vaccination trials that can be compared with each other.

It was also found that TNF-α by itself is an insufficient maturation stimulus, if apheresis cells are used as a starting population. Recently, it has been shown that MCM can be mimicked by a cocktail consisting of the pro-inflammatory cytokines TNF-α, IL-1-β, IL-6 and prostaglandin E2 (Jonuleit, H. et al., Eur. J. Immunol., 27, 3135 (1997)). These are the major constituents of MCM. Moreover, MCM was in most cases as effective as this cocktail, while the combination of TNF-α + Prostaglandin E2 (Rieser, C. et al., J. Exp. Med., 186, 1603 (1997)) is more variable. X-vivo 15 or 20 media supplemented with 1% autologous plasma have recently been recommended for the generation of fully mature DC (Jonuleit, H. et al., Eur. J. Immunol., 27, 3135 (1997)). However, it was found that RPMI medium was superior. Interestingly, the use of RPMI medium resulted in fully mature DC that expressed CD1a molecules, while DC generated in X-vivo media were CD1a negative or weakly positive. The adaption of the method for use with leukocyte apheresis products as a starting material obviates the need for repetitive blood drawings in order to generate DC and was established by Thurner, B. et al., J. Immunol. Methods 223, 1-15 (1999). Just a single leukapheresis is sufficient to generate DC for a whole series of vaccinations (≥ 5 x 10⁸ mature CD83+ DC / leukapheresis). DC are either repeatedly generated from frozen PBMC aliquots for successive vaccinations, or the whole apheresis product is processed at the onset in order to obtain a large number of DC that can be frozen in aliquots for later use using an optimized freezing protocol

However, a major drawback in the above methods is that the cultivation of the DC requires is generally performed in (a large number of) open vessels (wells or flasks). The various manipulation steps, i.e. the repetitive addition and removal of reagents, make it difficult that the whole procedure be processed under controlled and sterile conditions, i.e., a production method in accordance with GMP Guidelines is not yet available. Such GMP method is, however, a principal requirement for an *ex vivo* DC expansion or generation procedure (e.g., within the regimen of a DC-based vaccination) where the produced DC are intended to be retransfused to the original donator or another recipient.

Furthermore, there is the general need to facilitate the steps prior to the actual cultivation the dendritic cells, i.e. the isolation of suitable hematopoietic progenitopr cells from the patient.

### Summary of the Invention

The above problem has been solved with the methods and apparatus specified below. In particular, the present invention provides
(1) a method for producing mature and stable dendritic cells or immature dendritic cells according to GMP (Good Manufacturing Practice) guidelines which method comprises cultivating hematopoietic progenitor cells in a sterile cultivating apparatus in the presence of at least one dendritic cell differentiation/maturation factor, said cultivating apparatus comprising a closed container having an enlarged inner surface forming a grow area whereby the cells adhere to the surface;
(2) an apparatus for use in producing mature and stable dendritic cells which comprises a closed container having an enlarged inner surface forming a grow area; and
(3) a method for preparing peripheral blood mononuclear cells, which are suitable for cultivation of dendritic cells, from leukapheresis products, said method comprising lysis of the erythrocytes (and part of the granulocytes) within the leukapheresis product by the addition of water or aqueous ammonium chloride solution.

According to the invention the cells adhere to the enlarged inner surface of the container. To enlarge the inner surface of the container, the container may contain staple chambers and/or may be filled with particles, e. g. beads, spheres, spirals, sponge-like, wool-like or net-like structures, a three dimensional net, etc. which are made from polymeric material or alike. According to the plurality of these particles, the inner surface of the container is enlarged, whereby the cells adhere to the surface of these particles.

According to the invention, the particles can have any three-dimensional structure. It is, for example, possible to use nylon wool, ratings, spirals, sponge-like structures, and the like.

According to a preferred embodiment of the invention, the container comprises at least two fluidal communicating chambers, whereby each chamber defines a grow area. This kind of container has one or more separating walls separating the container into a number of chambers. According to the invention, the chambers are fluidal connected. Therefore it is possible to provide the apparatus with only one main inlet opening through which the fluid containing the cells to be cultivated can be inserted. Preferably, the fluid will be regularly divided between the chambers through fluidal communicating means.

Preferably, the communicating means are arranged between the chambers in a way that the container has to be held in a communicating state. Only in this state, the container fluid will be equally distributed between the chambers. If the container is held in a cultivating state or position, the chambers are not fluidal connected to each other so that fluid will not flow from one chamber to another in this state.

### Description of the Figures

Preferred embodiments of the apparatus according to the invention are shown in the figures, whereby
Fig. 1 shows a schematic top view of an apparatus according to the invention having stackable basin-like chambers,
Fig. 2 shows a cross section view of the apparatus shown in fig. 1 along the line X-X,
Fig. 3 shows a schematic top view of a second preferred embodiment of the apparatus according to the invention and
Fig. 4 shows a cross section view of the apparatus shown in fig. 3 along the line XII-XII.
Fig. 5 shows a cross-section view of a third preferred embodiment of the apparatus according to the invention and
Fig. 6 shows a cross-section view of a fourth preferred embodiment of the apparatus according to the invention.

### Detailed Description of the Invention

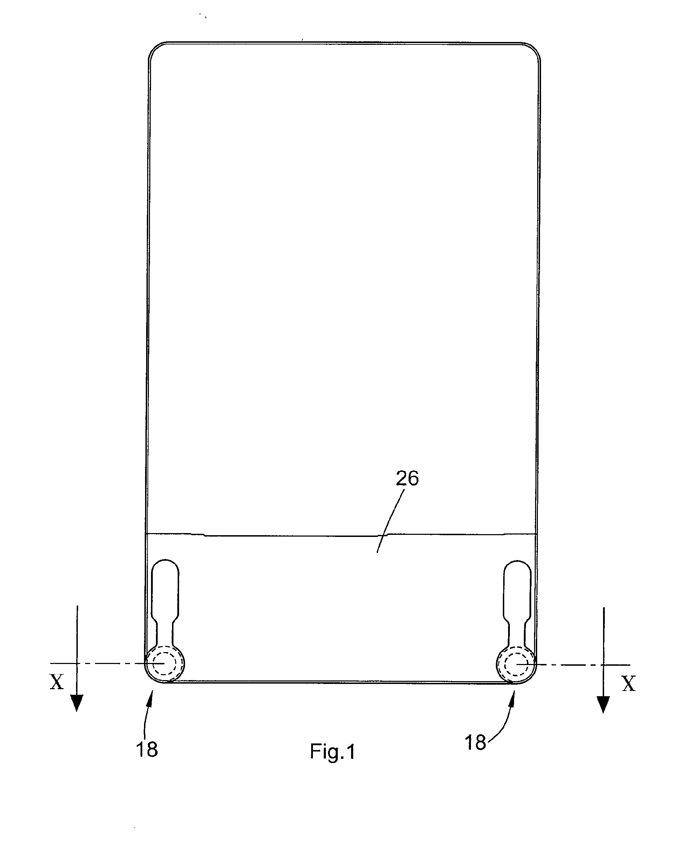

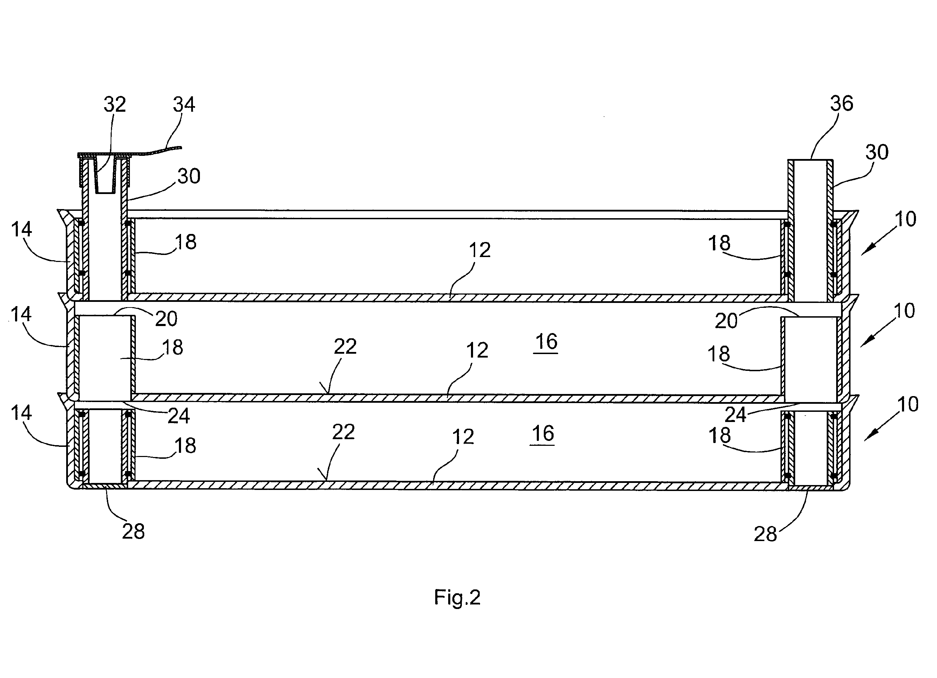

Figures 1 and 2 show the first preferred embodiment of the apparatus, which can be used for producing mature and stable dendritic cells or immature dendritic cells. The shown apparatus comprises stackable basin-like means 10, that are stackable as shown in figure 2. Each basin-like means 10 has a bottom part 12 and a side wall 14 surrounding the bottom part 12. By stacking one basin-like means 10 on top of another basin-like means 10, the bottom part 12 serves additionally as top part so that chambers 16 are formed by two basin-like means 10 stacked together, as shown in figure 2. The upmost basin-like means 10 forms the cover of the chamber 16 being below the bottom part 12 of the upmost basin-like means 10.

Each basin-like means 10 has at least one means 18 for connecting adjacent chambers 16. Within the shown preferred embodiment each basin-like means 10 comprises two means 18 for fluidal connection of adjacent chambers 16. The means 18 for fluidal connection are formed as preferably cylindrical hollow projections. In other words, the means 18 for fluidal connection are formed as a pipe or tube. Each of these tubes has an opening 20 being arranged in a distance to a bottom surface 22 of the bottom part 12. Opposite the opening 20, each tube has a second opening 24 arranged in the plane of the bottom part 12. This second opening 24 is open to the adjacent chamber 16.

Figure 2 shows the apparatus in a cultivating state or position. In this horizontal position the fluid is arranged on the bottom surface 22 whereby the cells adhere to the bottom surface 22. Therefore, the whole basin-like means 10 and at least the bottom part 12 are made from plastic materials including but not limited to polycarbonate, polystyrene and polyolefines. Alternatively, the bottom part 12 said materials can be coated by suitable adherence mediating agents such as an Ig (Immunoglobulin) coating (including IgG coating) or a coating with antibodies to cell surface molecules on monocytes (including CD14, CD16) so that the cells will adhere to the bottom surface 22.

In the shown cultivating state, each chamber 16 could be filled until the level of the fluid is higher than the opening 20 of the tubes 18. By using the shown apparatus for the described method, the level of the fluid will be much lower. The thickness of the fluid will be about 3 mm.

For the fluidal connection of the shown chambers 16, the apparatus shown in figure 2 will be rotated by 90 C° so that the tubes 18 are arranged on bottom (figure 1). Therefore fluid 26 will be equally distributed between the chambers 16 through the tubes 18.

On the openings 24 of the tubes 18 of the lowest basin-like means 10 are closed by covers 28. The upmost basin-like means 10 which is not used for cultivating has tubular parts 30 inserted in the tubes 18. One of the tubular parts 30 is closed by a cover 32 which is sterile closing the opening of the tubular part 30. The cover 32 is connected to a stripe 34 that can ripped of the tube 30. As shown in figure 2, the other tubular part 30 is already opened, i. e. the cover 32 was removed. Therefore, fluid can be filled in the chambers 16 through the opening 36 of the tubular part 30 forming the main inlet opening 36. The fluid is filled into any chambers through the main opening 36. While the fluid is filled in, the apparatus can be already held in its cultivative state. Each of the tubular parts 30 can be used as inlet and/or outlet opening for fluids. In the cultivating state the area above the fluid is filled in each chamber 16 with gas. This gas can be exchanged, for example, by pumping air with 5% CO₂ through the chamber 16.

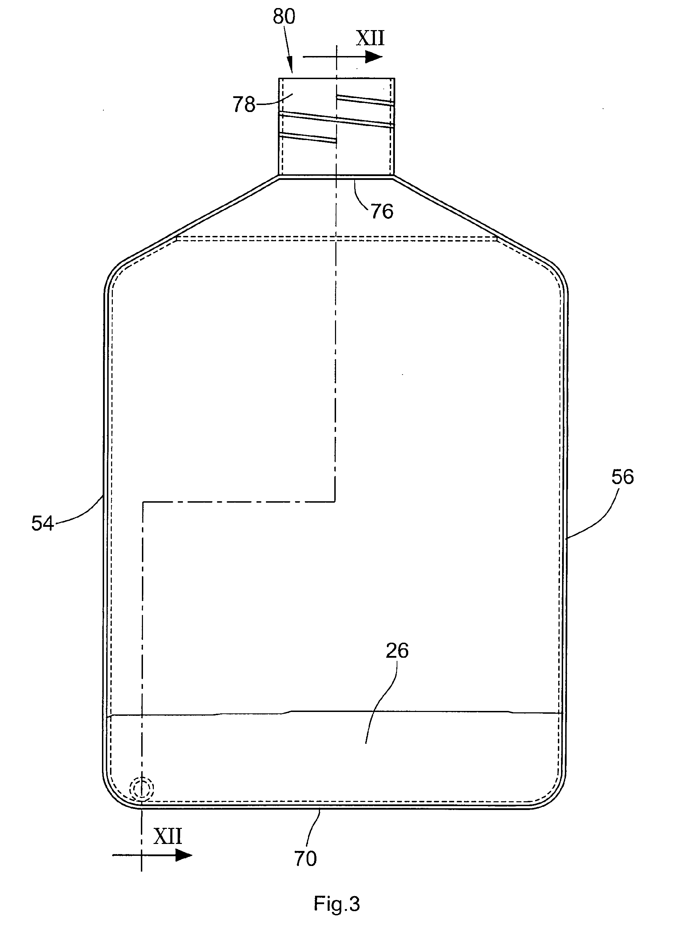

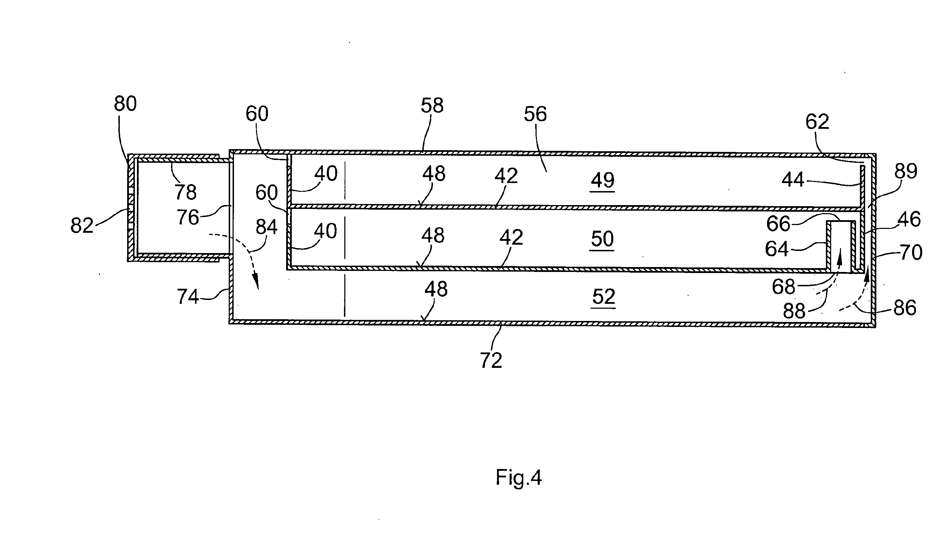

The second embodiment shown in figures 3 and 4 is a bottle-like container having separating walls 40, 42, 44 and 46 whereby these separating walls 40, 42, 44, 46 are forming three chambers 49, 50 and 52 (figure 4) in the shown embodiment. The number of chambers can also be higher. The upper chamber 49 is built by basin-like means having a bottom part 42 which is horizontally in figure 4 and side walls 40 and 44. The bottom part 42 has a surface 48 on which the cells adhere according to the inventive method. So that the fluid is held within the chamber 49 in the cultivating state or position as shown in figure 4 the bottom part 42 is sealably connected to side walls 54 and 56 extending on the total height of the container. The side walls 40 and 44 are also sealably connected to the walls 54 and 56. Next to a top wall 48, each side wall 40, 44 has an opening 60 and 62, respectively.

The middle chamber 60 has also a bottom part 42 being sealably connected to the side walls 54 and 56 and side wall 40. On the opposite side of the side wall 40, the middle chamber 50 has a side wall 46 without an opening. Therefore, the side wall 46 is sealably connected to the bottom part 42, the side wall 44 of the upper chamber 49 and the side walls 54 and 56. Additionally, the middle chamber 50 has a tubular projection 64 being sealably connected to the bottom part 42 and having an opening 66 being opened to the middle chamber 50.

Opposite the opening 66, the tube 64 has a second opening 68 in the plane of the bottom part 42 being opened in direction to the lower chamber 52.

The boundaries of the lower chamber 52 are formed by a side wall 70, a bottom wall 72 and a side wall 74 being opposite of the side wall 70. The side wall 70 extends from the bottom wall 72 to the upper wall 58.

Within the side wall 74, an opening 76 is formed. The opening 76 has a tubular projection 78 being closed by a cover 80 (figure 4). Within the cover 80, a hydrophobic filter 82 is arranged so that air can enter the container.

To fill the chambers 49, 50 and 52 with the same amount of fluid 26 the bottle-like apparatus is held as shown in figure 3 so that fluid can be filled in the chambers through the tubular projection 78 and the opening 76 in direction of the arrows 80. If the apparatus is held in this communicating state, the fluid flows in the chambers as shown in figure 4 by arrows in broken lines. First of all, the fluid flows in the direction of the arrow 84 in the lower chamber 52. From the lower chamber 52, the fluid flows in direction of the arrow 86 in a channel 89 which is connecting the lower chamber 52 with the upper chamber 48 through the opening 62 in the side wall 44. The channel 89 is build by the side wall 70 and the opposing side walls 46 and 44. Additionally, the fluid flows in the direction of an arrow 88 through the tube 64 and the opening 66 in the middle chamber 50. If the chambers 49, 50, 52 are equally filled with the same amount of fluid, the bottle-like apparatus will be rotated in the position shown in figure 4 so that the fluid is distributed equally over the flat bottom parts 42 and 72.
Suitable sterile cultivation apparatuses in accordance with Figs. 1 to 4 of the present invention are Nunc cell factories and Nunc triple flasks.

In a further embodiment of the invention, the enlarged inner surface (22, 48) of the container is formed by one or more regularly or irregularly shaped structures or particles, whereby the cells adhere to the inner surface of the container and/or to the surface of said particles. Suitable structures and particles are beads, spheres, spirals, rolled up foil or film, sponge-like wool-like or net-like structures, a three dimensional net , etc. as set forth above.

The aforementioned two preferred embodiments of the invention can also be used in a continuous or non-continuous fluid perfusion method for producing mature and stable dendritic cells. For this, the tubular parts 30 (Fig. 2) and the tubular projection 28 (Fig. 4) can be connected to a flexible tube or the like. The flexible tube is connected to a peristaltic pump or another device for transporting the fluid so that the fluid can be pumped in the chambers 16 and 49,50,52, respectively.

To distribute the fluid in the chambers, especially to distribute the fluid over the bottom surfaces 22,48 to which the cells adhere, the apparatuses can be moved or inclined.

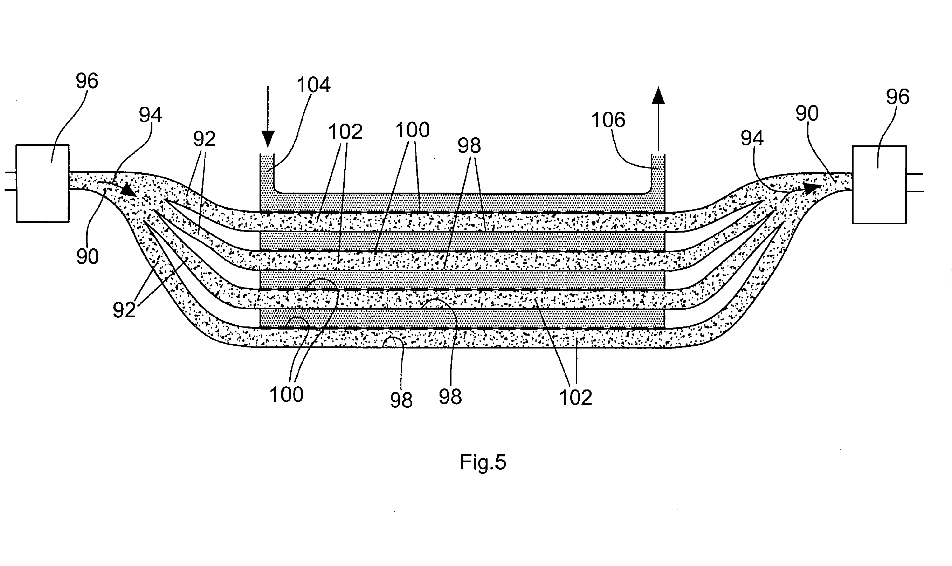

According to the third preferred embodiment (Fig. 5), the medium is pumped through a flexible tube 90. The tube 90 is separated into two chambers 92. Within the chambers 92 the cells adhere to a surface of these chambers or to particles that are arranged within the chambers 92. The fluid will be transported continuously or non-continuously in direction of the arrow 94. The two chambers are again connected to the tube 90 after the fluid has passed the chambers 92. To transport the fluid through the chambers 92, pumps 96, particularly peristaltic pumps, are used.

Each chamber 92 has a bottom surface 98. Opposite the bottom surfaces 98, air-permeable surfaces 100 are performed. These surfaces 100 are air-permeable, but fluid-non-permeable. Through the surfaces 100, gas can pass out of the chambers 92 into intermediate gas chambers 102, and vice versa. Each gas chamber 102 is connected to common inlet and outlet openings 104 and 106, respectively, so that gas can be supplied and dissipated from the gas chambers 102. The embodiment shown in Fig. 5 can particularly be used for non-continuous fluid supply.

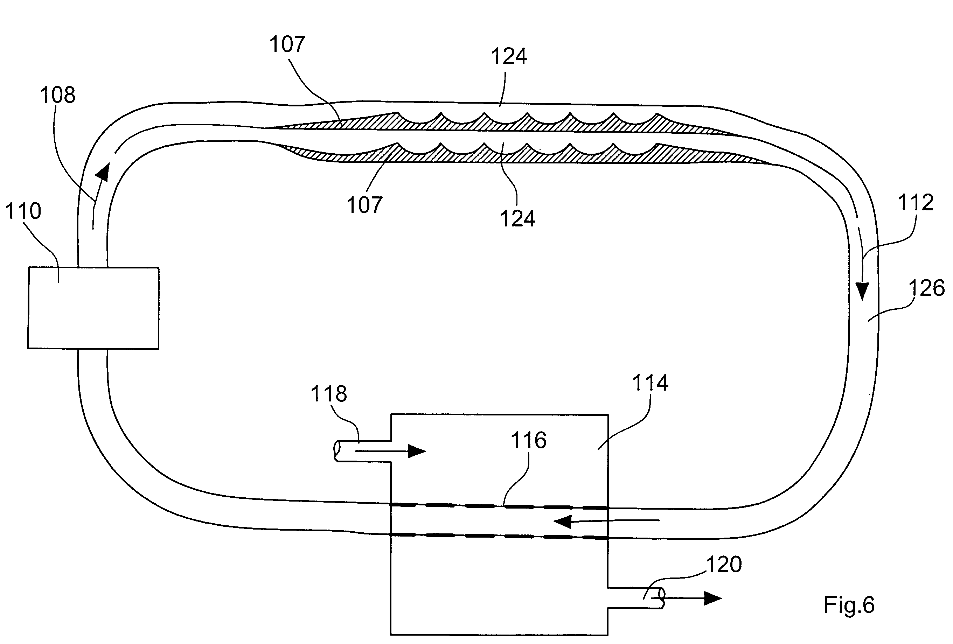

Another preferred embodiment (Fig. 6) is used for a continuous supply of fluid to two or more chambers 124. Each chamber 124 has a bottom surface 107 that has, for example, an enlarged surface by use of a wavelike structure. The two chambers 124 are connected to a common flexible tube 126. To supply the medium to the chambers 124 in direction of the arrow 108, a pump 110, particularly a peristaltic pump, is used. The medium flowing out of the chambers 124 in direction of the arrow 112 into the flexible tube 126 will be transported into a gas exchanging device 114. Within the gas exchanging device, the tube has a gas-permeable and a liquid non-permeable surface 116. Through the surface 116, gas can be exchanged between the flexible tube 126 and the gas exchanging device 114. Therefore, the gas exchanging device has an inlet opening 118 and an outlet opening 120.

To prevent the cells from being pumped into the tube 126 and the gas exchanging device 114, the chambers 124 can be tilted so that the cells will stay within the chambers 104 due to their weight.

The chambers of the container (apparatus) and/or the regularly or irregularly shaped particles and structures within the chambers preferably provide for an effective surface, i.e., a grow area of the inner surface of the container, of 25 to 10000 cm², preferably of 500 to 5000 cm². In accordance with the invention the are made from plastics, preferably polycarbonate, polystyrene or polyolefin. In a preferred embodiment of the invention, the inner surface of the container, preferably the grow area of the inner surface including the basin like means (10) and the surface of the regularly or irregularly shaped structure or particles, are coated with an adherence mediating agent (including agents that let monocytes adhere). Suitable adherence mediating agent are an Ig coating (including, but not limited to, an IgG coating), a coating with antibodies to cell surface proteins (including, but not limited to a coating with anti-CD14 or anti-CD16 antibodies), coating with adhesion molecules (including, but not limited to, selectins such as E-selectin and P-selectin, and/or members of the immunoglobulin superfamily such as VCAM-1) and the like.

The generation of the mature and immature dendritic cells (DC) of embodiment (1), i.e., the cultivation of the progenitor cells, is performed analogous to methods known in the art. Suitable progenitor cells are pluripotent cells, dendritic cell precursors or immature dendritic cells, preferably said hematopoietic progenitor cells are dendritic cell precursors which
(i) are obtained from CD14⁺ mononuclear cells (monocytes), from CD34⁺ cells, or directly from blood, and/or
(ii) are derivable from leukapheresis products, elutriation protocols, peripherally drawn blood or bone marrow.
For instance, the progenitor cells (and also the DC) are obtainable from leukapheresis products in accordance with the method disclosed in Thurner, B. et al., J. Immunol. Methods 223, 1-15 (1999) which is herewith incorporated in its entirety. Alternatively said progenitor cells can be isolated from the leukapheresis products by the method of embodiment (3) of the invention, which is further defined below. The cultivation is preferably performed by the following steps:
(i) applying peripheral blood mononuclear cells into the cultivation chamber and allowing them to adhere to the inner surface of the chamber,
(ii) washing the chamber in order to remove non-adhering cells,
(iii) cultivating the adhering cells in a culture medium in the presence of at least one dendritic cell differentiation/maturation factor, and optionally
(iv) adding further differentiation/maturation factor to the culture medium, and/or
(v) removing the culture medium, washing the adhering cells and cultivating the adhering cells in a culture medium comprising same or different dendritic cell maturation factors as compared to those of step (iii).

In a preferred mode of embodiment (1) of the invention the cultivation comprises
(i) loading 3x10⁵ to 4x10⁶ progenitor cells per cm² (effective) grow area, and/or
(ii) adding 0,01 to 3 ml, preferably 0,1 to 0,5 ml, culture medium per cm² grow area so that the grow area is sufficiently covered with culture medium, preferably from 1 to 5 mm. In particular, a Nunc triple flask (having a grow area of 500 cm²) is loaded with 150 to 2000x10⁶ cells in 20 to 200 ml culture medium and a Nunc cell factory (2 stacks; having a grow area of 1200 cm²) is loaded with 360 to 4800x10⁶ cells in 100 to 400 ml culture medium)

Suitable differentiation factors are mixtures of GM-CSF and IL-4, IL-13, IL-15 or IFN-α. Suitable dendritic cell maturation factors for the method of the invention include, but are not limited to, MCM, IL-1β, IL-6, TNF-α, PGE₂, IFN-α, lipopolysaccharides and other bacterial cell products (such as MPL (monophosphoryl lipid A) and lipoteichoic acid), phosphoryl choline, calcium ionophores, phorbol ester (such as PMA), heat-shock proteins, nucleotides (such as ATP), lipopeptides, artificial ligands for Toll-like receptors, double-stranded RNA (polyI:C), immunostimulatory DNA sequences, CD40 ligand, etc. The most preferred maturation factor is a mixture of IL-1β, IL-6, TNF-α, and PGE₂.

In the method of embodiment (3) of the invention, if the lysis is performed with water, it is preferred that
(a)the lysis is performed at 20 to 30 °C for 5 to 30 s; and/or
(b) the volume ratio leukapheresis product to water is 20:1 to 2:1.
On the other hand, if the lysis is performed with aqueous ammonium chloride, it is preferred that
(a)the concentration of the ammonium chloride in the added solution is 0,5 to 5 % (w/w) preferably 1 to 3 % (w/w); and/or
(b)the lysis is performed at 25 to 50 °C for 5 to 20 min; and/or
(c) the volume ratio leukapheresis product to ammonium chloride is 20:1 to 2:1.
The above method may further comprise the steps quenching and/or washing and/or suspending the lysis product within a suitable culture medium (without plasma or proteins) or salt solution (such as slightly hypotonic salt solutions).

In particular, in the lysis with water the leukapheresis product is filled into 50 ml tubes (10 ml per tube) and centrifuged (1100 rpm, 4°C 12 min). The supernatant is discarded, 20 ml Aqua dest. (5 to 30 ml)) are added to the pellet, which is then resuspended, vortexed and after 20 seconds (5 to 30 s) filled up with 30 ml complete medium, preferably supplemented with 800 U/ml GM-CSF. The suspension is then washed as described in Thurner, B. et al., J. Immunol. Methods 223, 1-15 (1999). Lysis can also be performed in larger vessels, for example, the leukapheresis product in a leukapheresis pouch and spinned (1100 rpm, 4°C, 12 min). The supernatant is discarded, 50 ml of Aqua dest. is added, the pellet is resuspend and after 20 s (5 to 30 s) filled up with 150 ml complete medium and proceeded with washing steps as described above.
In the lysis with ammoniumchloride, the leukapheresis product is filled into 50 ml tubes (10 ml per tube) and centrifuged (1100 rpm, 4°C, 12 min). The supernatant is discarded. The pellet is resuspended with 10 ml complete medium supplemented with 800 U/ml GM-CSF and 10 ml (5 to 20 ml) 1,6 % NH₄Cl is added. The suspension is shaken well, put into a water bath (37°C) for 10 (5 to 15) min and proceeded with washing steps as described above. Again this lysis procedure can be adapted to larger vessels as the lysis with water described above.

The invention is further illustrated by the following, non-limiting examples.

### Examples

### Materials and Methods:

All reagents and materials employed in the protocol that was developed to generate DC for clinical application were endotoxin-free, and most were in GMP (Good Manufacturing Practice) quality as far as available.

Equipment: microbiological safety workbench (Heraeus, HERA safe HS 12/2); centrifuge (Heraeus, Megafuge 2, ORS); incubator (Heraeus, Cytoperm 2); - 80 °C freezer (National Lab, Profimaster EPF); -20 °C freezer (Liebherr, GS 1382); refrigerator Liebherr KB 1001 (Liebherr, Berlin); transmitted light microscope (Leica, DMLS); reflective light microscope (Leica, DMIL); pipetting aid Pipet-aid XP (Drummond); pipettes, Eppendorf Reference (Eppendorf); Neubauer counting chamber (Superior Marienfeld)
Plastic material: 50 ml tubes, conical polypropylene tubes, sterile due to gamma irradiation (Corning Costar, Product No. 430829); 15 ml tubes, conical polypropylene tubes, sterile due to gamma irradiation, 25 tubes/bag, (Corning Costar, Product No. 25315-15); disposable pipettes, pyrogen-free, individually packaged, sterile due to gamma irradiation (Corning Costar, Product No. 4485/1 ml, 4486/2 ml, 4487/5 ml, 4488/10 ml, 4489/25 ml); pipette tips, sterile and individually packaged (Biozym, Safeseal-Tips, Product No. 790011 /10 µl, 790101/100 µl, 791001/1000 µl); disposable sterile filters, 0.22 mm, sterile and endotoxin-free, individually packaged (Millipore, Millex-GS, Product No. SBGS025SB); culture dishes with cell culture surface, NUNCLON® surface products [Nunc Cell factory, stacked by two, individually packaged, original package, sterile due to gamma irradiation, endotoxin-free (Nunc, Prod. No. 167695); cell culture jars Nunc Triple Flask, sterile due to gamma irradiation, endotoxin-free (Nunc, Prod. No. 1328867 or 132913)]; 6-well cell culture plates, individually packaged, Optilux surface, sterile due to gamma irradiation, endotoxin-free (Falcon/Becton Dickinson, Product No. 3046); air filter Midisart 2000 (Sartorius, Prod. No. 17805-G); freezing tubes, sterile, endotoxin-free (Nunc, Nunc Cryo Tube vials, Product No. 375418/1.8 ml, 337516/4.5 ml); cannulas: Stericam 0.9 x 70 mm 20G x 2 4/5 Luer Thin-Walled Disposable Cannulas, (Braun, Product No. 04665791); Microlance 3 0.4 x 19 mm 27G 1 3/4 REF 302200 (Becton Dickinson); sterile surgical gloves Peha-taft made of latex (Hartmann, Prod. No. 9423 53/8 (size 7) or 9423 54/7 (size 7 1/2)); sterile surgical gloves Peha-taft Syntex, latex-free (Hartmann, Prod. No. 942633/8 (size 7)).

Chemicals: Molgramostim (GM-CSF) (Leukomax® 400), 54.38 mg of dry substance and 1 ml of sterile H₂O, Medical Drug Approval No. 25756.03.00) (Sandoz, Product No. PZN-4608744); NaCl 0.9%, 10 ml vials (Braun, Prod. No. 02246228); aqua ad injectionem, 10 ml vials (Braun, Prod. No. 02240246); PBS, sterile and endotoxin-free, GMP quality (Bio Whittaker/Serva, Product No. 17-512F); recombinant human IL-4, sterile and endotoxin-free, GMP quality (Cell Genix); interleukin 1b (Amedak); interleukin 6 (Novartis); tumor necrosis factor α (Böhringer Ingelheim); prostaglandin E2 Minprostin® (Pharmacia); 70% alcohol (Laborcenter, Nürnberg); Barrycidal® 36, disinfectant (Helmut Schröder) - Incidin Plus® (Henkel); sterile sponges Gazin, sterile and individually packaged (Lohmann, Product No. 0197); trypan blue 0.4% sterile (Sigma Prod. No. T-8154).

Culture medium, cytokines, monoclonal antibodies (mAbs): The following culture media were used: as standard medium RPMI 1640 (Prod. Nr. 12-167, Bio Whittaker, Walkersville, USA) supplemented with gentamicin (Refobacin 10, Merck, Darmstadt, Germany)at 20 µg/ml final concentration, glutamine at 2mM final concentration (Prod. Nr. 17-605, Bio Whittaker) and 1% heat inactivated (56° for 30 min) human plasma was used [= further on called complete medium]. Human plasma was either autologous heparinized (500 I.U./20ml blood, Liquemin N 2500, Hoffmann La Roche, Basel, Switzerland) plasma (obtained from freshly drawn blood) or 10% ACD-A (acid-citrate-dextrose Formula A, Fresenius AG, Bad Homburg, Germany) plasma (yielded by leukapheresis procedure) or for selected experiments single donor allogeneic AB positive ACD-plasma obtained from the Department of Transfusion Medicine.

Alternatively, we tested X-VIVO 15 and X-VIVO 20 (Bio Whittaker) supplemented the same way as our standard medium.

Recombinant human GM-CSF (800 U/ml) in GMP quality (Leukomax® Sandoz, Basel, Switzerland) and recombinant human IL-4 (500 U/ml) (kindly provided by Dr. E. Liehl, Novartis Research Institute, Vienna, Austria) were used for the standard cultivation procedure. Human IL-4 obtained from Genzyme Corporation (Cambridge, Massachusetts, USA) - produced according to GLP (Good Laboratory Practice) and tested according to GMP guidelines - was also used.

In those experiments comparing MCM with maturation inducing cytokines, recombinant human 10 ng/ml TNF-α (kindly provided by Dr. Adolf, Bender & Co, Vienna, Austria), 1000 U/ml IL-6 (kind gift of Novartis, Basel, Switzerland) , 10 ng/ml IL-1β (Sigma, St. Louis, MO USA) and 1µg/ml prostaglandin E2 (Cayman Chemical, Ann Arbor, MI, USA) was used. For IL-10 resistance experiments, rh IL-10 in a dosage of 10 ng/ml (Genzyme Corporation, Cambridge, Massachusetts, USA) was used.

For flow cytometry, monoclonal antibodies against the following antigens were used: CD1a (Ortho Diagnostic System, Germany), CD2, CD4, CD8, CD14, CD19, CD25, CD56, HLA-DR (all obtained from Becton Dickinson, Brussels, Belgium) CD3, CD40, CD86, (Cymbus, Dianova, Hamburg, Germany), CD83 (Immunotech, Marseilles, France), CD95 (Pharmingen, San Diego, USA), CD 115 (Calbiochem, Massachusetts, USA). Isotype controls were run in parallel.

For intracellular FACS staining, cells were fixed and permeabilized with Fix & Perm (Biozol, Eching, Germany) according to the manufacturer's instructions, then stained with p55 / anti-fascin supernatant (Mosialos, G. et al., Am. J. Pathol., 148, 593 (1996)) (K-2 clone, kindly provided by Dr. E. Langhoff, Boston). As secondary antibody fluorescein-conjugated AffiniPure F(ab')₂ goat anti mouse IgG, Fc gamma fragment specific (Jackson Immuno Research, Dianova, Hamburg, Germany) was used.

Leukocyte apheresis and isolation of PBMC: Initial processing of apheresis products: Leukapheresis products were obtained from the Department of Transfusion medicine as monocyte separation products from healthy donors or melanoma patients after informed consent was given (the healthy volunteers were regular cytapheresis donors, the melanoma patients were treated in the course of phase I DC vaccination trials that were approved by the ethical committee of the University of Erlangen-Nuerenberg as well as by the international review board of the Ludwig Institute for Cancer Research, New York, USA). As cell separators either Cobe Spectra (Cobe BCT, Inc., Lakewood, CO, USA) or Fresenius ASTEC 204 (Fresenius AG, Bad Homburg, Germany) were used. Cobe spectra was used with the white blood cell set and the MNC program, Fresenius ASTEC 204 with the P1Y-Set and the MNC program. During the leukapheresis, acid-citrate-dextrose Formula A (ACD-A, Fresenius) was used as an anticoagulating substance following the manufacturer's instructions. After dilution (leukapheresis product is filled into a 600 ml culture flask by using a perfusor syringe, then PBS containing 10% ACD-A is added to a final volume of 480ml) with PBS (Prod. Nr. 17-512, Bio-Whittaker, Walkersville, USA)/10% ACD-A (Fresenius AG), the PBMC were isolated by centrifugation in Lymphoprep (1.077 g/ml; Nycomed Pharma, Oslo, Norway) at 460 g and room temperature for 30 minutes (15 ml lymphoprep are overlayed with 30 ml diluted leukapheresis product). Cells were washed three times in PBS without calcium or magnesium containing 1 mM EDTA (Bio Whittaker), starting with the first centrifugation at 250 g, the second with 175 g, and the third with 110 g , each for 12 minutes at 4°C.

Generation of autologous monocyte conditioned medium (MCM): Ig coated bacteriological plates (85 mm, Falcon 1005) were prepared immediately prior to use. As immunoglobulin we used Sandoglobin™ (Novartis, Basel, Switzerland). Coating was performed with 10 ml of diluted ( with PBS without calcium or magnesium, Bio Whittaker) immunoglobulin (10µg/ml) for 10 minutes at room temperature. After the coating procedure plates were rinsed twice with PBS without calcium or magnesium (Bio Whittaker). 50 x 10⁶ PBMC were plated on these dishes in complete medium without cytokines and incubated at 37°C, 5% CO₂ for 20 hours. Then the monocyte conditioned medium was harvested, centrifuged at 1360 g for 10 min (22°C), sterile filtered (0,22 µm filters, Millipore, Molsheim, France) and frozen down in aliquots at -20°C. In the case of monocyte conditioned medium, 750µl of MCM were added per well (containing 3 ml volume), i.e. 20 v/v %. Alternatively we added a cocktail of cytokines (IL-1β + IL-6 + TNF-α, each at 10ng/ml) or TNF-α alone (10-20-40 ng/ml). Each maturation inducing stimulus was tested in combination with or without prostaglandin E2 (1µg/ml). See also Culture medium, cytokines.

Isolation of CD14+ monocytes by magnetic cell sorting (MACS): CD14+ cells were separated by performing a positive selection with CD 14 micro magnetic beads (Miltenyi, Bergisch-Gladbach, Germany) (Miltenyi, S. et al., cytometry, 11, 231 (1990)) according to the manufacturer's instructions.

### Alternative metods for the isolation of monocytes

A) Lysis with Aqua dest: The leukapheresis product was filled into 50 ml tubes (10 ml per tube) and centrifuged (1100rpm, 4°C, 12 min). The supernatant was discarded, 20 ml Aqua dest. was added to the pellet, was resuspended by vortexing for 20 seconds and was then filled up with 30 ml complete medium supplemented with 800 U/ml GM-CSF. The suspension was then spun with 1100 rpm at 4°C for 12 min, the supernatant was discarded and the remainder was filled up with 50 ml PBS/EDTA (1mM). The resulting suspension was spun with 900 rpm at 4°C for 12 min, the supernatant was discarded and the remainder was filled up with 50 ml PBS/EDTA . 10µl of cell suspension were taken out for counting of cells. The suspension was spun with 800 rpm at 4°C for 12 min, the supernatant was discarded and the remainder was filled up with complete medium (Complete medium: 500 ml RPMI 1640, 5 ml autologous heat inactivated plasma, 5 ml glutamin and 200µl gentamycin) and plated into/charged to the culture vessels in the usual manner.
B) Lysis with ammoniumchloride: The leukapheresis product was filled into 50 ml tubes (10 ml per tube) and centrifuged (1100rpm, 4°C, 12 min). The supernatant was discarded, the pellet was resuspended with 10 ml complete medium (supplemented with 800 U/ml GM-CSF) and 10 ml 1,6% NH₄Cl was added. The resulting suspension was shaken well and put into water bath (37°C) for 5 to 15 minutes. Thereafter the suspension was spun with 1100 rpm at 4°C for 12 min, the supernatant was discarded and the remainder was filled up with 50 ml PBS/EDTA (1mM). The suspension was then spun with 900 rpm at 4°C for 12 min, the supernatant was discarded and the remainder was filled up with 50 ml PBS/EDTA . 10ìl of cell suspension were taken out for counting of cells. The suspension was spun with 800 rpm at 4°C for 12 min, the supernatant was discarded and the remainder was filled up with complete medium and plated into/charged to the culture vessels in the usual manner.

Flow Cytometry: Cell populations were phenotyped with the panel of mAbs listed above and analysed on a FACScan (Becton-Dickinson) as described in Romani et al., J. Immunol. Methods, 196, 137 (1996). Dead cells were gated out on the basis of their light scatter properties.

T cell stimulatory/functional assays; Primary allogeneic MLR: To test T cell stimulatory function, DC were added to allogeneic T-cells in graded doses and coincubated for 4 to 5 days in RPMI 1640 supplemented with gentamicin, glutamine and 5% allogeneic heat-inactivated human serum (single donor). Tests were performed in 96 well flat bottomed plates with 2x10⁵ T-cells/well. T-cells were isolated by using separation columns according to the manufacturer's instructions, (TEBU, Frankfurt, Germany). Proliferation was determined by addition of 50 µl 3H Thymidine (4 µCi final concentration/ml) for 12 - 16 hs to triplicate wells.

T cell stimulatory/functional assays; Secondary allogeneic MLR: Naive CD4 T-cells were isolated with the CD4/CD45RA-muitisort-kit (Miltenyi, Bergisch-Gladbach, Germany) according to manufacturer's instructions. Purity of CD4+/CD45RA+ cells was ≥ 95%. Naive T cells (3x10⁶ / well) were seeded into macrowells (24 well plates, Falcon) in RPMI 1640 supplemented with gentamicin, glutamine and 5% heat-inactivated allogeneic human serum (single donor) and stimulated with mature DC (3x10⁵ / well). About three days later T cells were expanded with IL-2 (Proleukin, Chiron, Emeryville, USA) (50 U/ml). T-cells were restimulated two weeks after primary stimulation with mature DC generated from the same donor as for primary stimulation and under identical conditions. For restimulation 3 x 10⁶ T cells + 3 x 10⁵ DC were seeded into 24 wells. 48 h thereafter supernatants were harvested, frozen at -20°C, and later cytokines were measured by ELISA assays as described below.

Induction of influenza virus specific CTL; Preparation of T cells: T lymphocytes were enriched using rosetting with neuraminidase treated-sheep red blood cells as described (Bender et al. 1996)

Induction of influenza virus specific CTL: Mature DC prepared from HLA-A2.1 positive donors were washed and resuspended in RPMI 1640 to 0.5 - 1 x 10⁷ cells/ml. Live influenza virus was added at a final concentration of 1000 HAU/ml or DC were loaded with 50µg/ml influenza matrix peptide (IMP) GILGFVFTL for 1 h at 37°C . Cells were washed 3 times and 3 x 10⁴ DC were added to 1 x 10⁶ purified T cells in 24 well plates (Falcon). After 7 days of culture, the T cells were harvested and tested for cytolytic activity using an Europium release assay.

Measuring cytolytic activity: 3 x 10⁶ target cells (HLA A2.1+, MHC class II negative T2 cells) were washed and incubated with or without 50µg/ml influenza matrix peptide for 1 h at 37°C. After washing, 1 x 10⁶ T2 cells were incubated with 3 µl fluorescence enhancing ligand (BATDA, Wallac, Turku, Finland) in 1 ml culture medium supplemented with 10% FCS for 15 min at 37°C. Cells were washed at least 5 times in PBS and resuspended carefully in 10% FCS culture medium. 5 x 10³ target cells were incubated for 2 h at 37°C with effector cells in flat bottom 96 well plates. Effector : target ratios were 45:1, 20:1, and 5:1. After 2 h the 96 well plates were centrifuged, each supernatant was transferred in new 96 well plates and analyzed with a 1420-002 Victor TM multilabel counter (Wallac, Turku, Finland).

Measuring of endocytic activity and processing / presentation of TT: In a few experiments FITC dextran uptake was determined exactly as described by (Sallusto et al. 1995). Antigen processing / presentation activity of tetanus toxoid was tested using the tetanus-toxoid-peptide specific T cell clone AS11.15 (kind gift of Dr. Lanzavecchia, Basel Institute of Immunology, Basel) exactly as described (Romani et al. 1996).

Cytokine and PG E2 ELISA: For ELISAs, microtiter plates (Nunc Maxisorb II round bottom) were coated with antibodies specific for IL-6 (PharMingen) and IL-1β, IL4, Interferon-γ, TNF-α (all obtained from Endogen, Woburn, USA) over night at 4°C. The plates were washed and blocked with 1% human serum. Samples and standards were analysed in triplicate, and assayed with the avidin-peroxidase system. For Prostaglandin E2 ELISA, a ready to use kit (Amersham Pharmacia, Buckinhamshire, UK) was used. Plates were measured in an ELISA-reader (Wallac, Turku, Finland) at 405 nm wavelength.

Cryopreservation of PBMC or DC: PBMC were frozen using a fully automatic freezing unit (Nicool Plus, Air Liquid, Bussy-Paris, France) (starting point +6°C, endpoint -120°C, Tₓ -40°C) in freezing medium consisting of 20% GMP quality human serum albumin (Centeon, Marburg, Germany) + 10% (v/v final concentration) DMSO (Sigma, St. Louis, USA; Cat.No. 2650) at 10-35 x 10⁶ PBMC/ml freezing medium. Frozen volumes did not exceed 4,5ml / vial. Frozen cells were thawed in a 56°C heated water bath, then dumped into 5 ml of cold Hanks balanced salt (Bio Whittaker, Walkersville, USA), and centrifuged once for 10 minutes at 125g, 4°C. After that, PBMC were plated as described above. For freezing of DC at day 5 or 7 of culture we reduced cell density to 5 -15 x 10⁶ DC/ml, but the freezing medium and freezing procedure remained unchanged.

### Example 1 (Comparative Example): Generation of DC in Open Vessels

Generation of DC from PBMC: According to the method disclosed in Thurner, B. et al., J. Immunol. Methods 223, 1-15 (1999) PBMC were plated in 85 mm dishes (either bacteriological, Primaria or Tissue culture dishes, Falcon, Cat.No. 1005, 3038 or 3003; Becton Dickinson, Hershey, USA) at a density of 50 x 10⁶ cells per dish in 10 ml of complete culture medium and incubated at 37°C and 5% CO2 for 1 hour. After a microscopic control of adherence, the non-adherent fraction was removed and 10 ml of fresh, warm complete medium were added (day 0). The non- adherent fractions were centrifuged and plated once more in new 85 mm tissue-culture-dishes for readherence. The non-adherent fraction from these "replate" dishes was discarded after 1 h adherence. All adherent fractions were cultured until day 1, then culture medium was taken off carefully so that loosely adherent cells were not removed, and new culture medium containing GM-CSF (800 U/ml final concentration) and IL-4 (1000 U/ml final concentration) was added. Cytokines were added again on day 3 in 3 ml fresh medium (containing 8000 U GM-CSF and 10000 U IL-4) per dish. On day 5 all non-adherent cells were harvested, counted and replated in fresh complete medium (containing cytokines in the same dosage as described above) in 6 well plates at a density of 5 x10⁵ cells/well in 3 ml medium. On day 6 different stimuli to induce maturation of DC were added, and on day 7 or 8 (and in pilot experiments also days 9 and 10) cells were harvested.
Mature DC obtained by said method have the following properties (see Thurner, B. et al., J. Immunol. Methods 223, 1-15 (1999)):
(a) are non-adherent, veiled cells that remain morphologically stable upon removal of cytokines and further culture for 36 hours;
(b) display the characteristic phenotype of mature DC as determined by cytoflurometric analysis;
(c) display strong allostimulatory capacity in the primary allogeneic MLR at DC : T ratios of 1 : ≥ 300, and are resistant to the inhibitory effects of IL-10 (while immature DC (generated in GM-CSF + IL-4 but without exposure to maturation inducing MCM) lack these properties);
(d) induce strong cytolytic T cell responses;and
(e) display CD1a surface expression (while most DC generated in X-vivo 15 or X-vivo 20 media lack surface CD1a molecules though otherwise exhibiting a comparable phenotype).

### Example 2: Preparation of dendritic cells in nunc cell factories

### A.: Protocol for the preparation of human dendritic cells from fresh PBMCs in Nunc cell factories

1. Plating of PBMCs on day 0: Depending on how many PBMCs were obtained, a corresponding number of tissue culture vessels can be charged. For each Cell Factory tissue culture dish, 1200 x 10⁶ PBMCs each were plated in 200 ml each of complete medium (e.g., if you have 3800 million PBMCs: 1200 million x 3 = 3600 million; plate in 3 Cell Factories, store the rest by freezing as PBMC). The cells to be plated were transferred to a 50 ml tube and centrifuged once more (4 °C, 10 minutes, 700 rpm/110 x g).
   The supernatant was removed using a vacuum pump, the pellet was taken up with 10 ml of culture medium per Cell Factory to be plated and resuspended (= cell suspension). Per Cell Factory labeled ("name of patient"), 190 ml of medium were charged and 10 ml of cell suspension per Cell Factory were added. After carefully swinging the dishes, they were allowed to equilibrate between the levels and put into an incubator for 1 h.
2. Removal of non-adherent fraction after one hour, change of medium: After 60 minutes it was checked under a reflected light microscope whether a sufficient (about 70% of the dish surface should be covered by firmly adhering longish cells) adherence of the cells to the culture dish had been achieved.
   When the adherence was sufficient, the non-adherent fraction was removed by carefully agitating the cell culture vessels and the medium was poured off with the non-adherent cells into a sterile cell culture jar. The cell culture vessels were charged again with 140 ml of pure RPMI, agitated again, and poured off again. The process was repeated again, 200 ml of complete culture medium were added, allowed to equilibrate, and a new sealing ring and air filter were applied. 10 ml from the poured-off culture medium were removed using a 10 ml syringe and injected into a blood culture jar. The jar was labeled and immediately placed into an incubator, followed by a bacteriological check.
   Only one dish at a time was processed under the workbench. Then, the dishes were again placed into the incubator for 24 h.
   When the adherence was not sufficient, the dishes were left in the incubator for another 15 to 30 minutes before starting the removal of the non-adherent fraction. The non-adherent fraction was collected in 50 ml tubes and was again centrifuged (900 rpm/175 x g, 10 min, 18 °C). The collected non-adherent fraction of each Cell Factory was again plated in 1 Nunc Triple Flask in 100 ml of complete medium as described above (re-adherence), and incubated again at 37 °C for 60 minutes. The non-adherent fraction was again removed as described above (non-adherent cells can be discarded outside the GMP Department or frozen for immunomonitoring).
3. Addition of cytokines on day 1: GM-CSF preparation: Prewarmed original piercing jar Leukomax® 400 was dissolved with enclosed NaCl (1 ml) and diluted with 110 ml of PBS/2% human serum albumin (99 ml of PBS + 11 ml of 20% HSA, supplied by Behring). Subsequently aliquots were placed into 100 sterile 1.5 ml freezing tubes and stored at -80 °C.
   IL-4 preparation: Dry powder in original vials (Genzyme IL-4, 4 mg) was brought to room temperature, dissolved with 100 µl of aqua ad inject., diluted with 500 µl of PBS 2% HSA. Aliquots were placed into sterile 1 ml freezing tubes of 50 µl each and stored at -80 °C.
   For further use, IL-4 aliquots were diluted with 450 µl each of RPMI per vial.
   Culture dishes were carefully removed from the incubator and checked by reflected light microscopy. For each Cell Factory, 20 ml of culture medium plus 4800 µl each of GM-CSF and 180 µl each of IL-4 (for a total of 240 ml) was prepared, for each Triple Flask, 10 ml of culture medium plus 2000 µl each of GM-CSF and 75 µl each of IL-4 (for a total of 100 ml) was prepared (corresponding to 800 U/ml GM-CSF and 500 U/ml IL-4). The new medium was added and allowed to equilibrate. Subsequently, the cells were transferred to the incubator and maintained into the incubator again for 48 h.
4. Addition of cytokins on day 3: The required amounts of GM-CSF and IL-4 were thawed. The culture medium was stored in a warm place. For each Cell Factory, 40 ml of culture medium plus 6000 µl each of GM-CSF and 225 µl each of IL-4 (for a total of 300 ml), for each Triple Flask, 20 ml of culture medium plus 2600 µl each of GM-CSF and 97.5 µl each of IL-4 (for a total of 130 ml) was prepared (corresponding to 800 U/ml GM-CSF and 500 U/ml IL-4).
   The culture dishes were carefully removed from the incubator and checked by reflected light microscopy. The new medium was added and allowed to equilibrate. Subsequently, the cells were transferred into the incubator again for 48 h.
5. Addition of cytokins on day 5: The required amounts of GM-CSF and IL-4 were thawed. The culture medium was stored in a warm place. For each Cell Factory, 40 ml of culture medium plus 6000 µl each of GM-CSF and 225 µl each of IL-4 (for a total of 300 ml), for each Triple Flask, 20 ml of culture medium plus 2600 µl each of GM-CSF and 97.5 µl each of IL-4 (for a total of 130 ml) was prepared (corresponding to 800 U/ml GM-CSF and 500 U/ml IL-4).
   The culture dishes were carefully removed from the incubator and checked by reflected light microscopy. New medium was added and allowed to equilibrate. For distributing the cells, the dishes were carefully swung and again transferred into the incubator.
6. Addition of maturing cocktail on day 6: The required amount of cytokins was thawed (10 µl of maturing cocktail per ml of culture medium) at room temperature under the running workbench. The maturing cytokins were in a dissolved form and concentrated such that the addition of 10 µl of maturing cocktail per ml complete medium corresponds to the concentrations stated below.
   The final concentrations of the individual cytokins/ml correspond to:
   - 2 ng: IL-1β (interleukin 1β)
   - 1000 U: IL-6 (interleukin 6)
   - 10 ng: TNF-α (tumor necrosis factor α)
   - 1 µg: PG E2 (prostaglandin E2)
   Cell Factories contain:
   - 355 ml: i.e., 3.55 ml of maturing cocktail per Cell Factory
   Triple Flasks:
   - 156 ml: i.e., 1.56 ml of maturing cocktail per Triple Flask
   The vessels were removed from the incubator and evaluated under a reflected light microscope. The maturing cocktail was pipetted into the dishes in the amounts stated above, were allowed to equilibrate and were retransferred into the incubator.
7. Harvesting of the mature dendritic cells on day 7:
   If the bacteriological checks of the culture media of d0 and d5 are satisfactory, i.e., the samples are found to be sterile, the following optical check of the DCs will be performed:
      The vessels were removed from the incubator and the surfaces with the dendritic cells were evaluated under a reflected light microscope. A polaroid photo was prepared using the reflected light microscope.
   If the cells have a healthy appearance (hardly any dead cells, hardly any adherence, culture medium was red and clear, no visible microorganisms, no sign of contamination, please see also SOP DC 12, item 1), 4 ml of cell suspension is removed from a Cell Factory and used to perform a FACS analysis.
   If the FACS staining yields a sufficient CD 83 expression (> 75% CD 83 positive), the cells can be harvested. If CDE 83 expression is not yet sufficient (< 75%), it is necessary to wait for another 3 hours before a new FACS analysis is performed.
   For harvesting of the cells the contents of the Cell Factories and of the Triple Flasks were transferred to 50 ml tubes. The cell culture vessels are rinsed another two times with 100 ml each of RPMI. Centrifugation at 700 rpm/110 x g, 12 min, 4 °C.
   For freezing the cells, autologous serum with 20% DMSO (alternatively, 20% human serum albumin with 20% DMSO and 5% glucose?) was prepared (e.g., 36 ml of serum with addition of 4 ml of DMSO). The freezing medium was cooled on ice.

A reference tube (3.6 ml) was charged half (1.8 ml) with freezing medium and half with pure serum (alternatively, 20% HSA), so that the final concentration of DMSO was 10%. The freezing unit was started.

For a repeated bacteriological check, 10 ml of the supernatant was transferred to a blood culture jar. The cells were taken up with 40 ml of culture medium, followed by removing 40 µl for cell counting. 10 µl of cell suspension (to which 10 µl of trypan blue was added) were pipetted into a Neubauer chamber and cells were counted according to the manufacturer's instructions.

One million DCs were required for further quality control tests, another million DCs were frozen as a lot control in an additional 1 ml vial.

The cells were concentrated in pure autologous serum (alternatively, 20% HSA) in a concentration of about 20 million/ml and cooled on ice. Previously labeled freezing tubes were cooled on ice and each of them was charged half with the cooled cell suspension (e.g., in 3.6 ml tubes, 1.8 ml each of cell suspension). Once the freezing unit was ready for inserting the tubes, the freezing medium was added to the cooled cell suspension, the tubes were sealed with threaded caps, swung and placed into a freezing unit, and the freezing process was started.

After completion of the freezing process (reaching of -130 °C), the tubes were transferred into liquid nitrogen.

### B: Results

With the above Protocol a DC yield of 9.26% could be achieved (average value out of 19 patients leukapherese products). The CD 83 Expression was 89.53%. The obtained DCs showed identical properties as compared to the DC of Example 1.

## Claims

**1.** A method for producing mature and stable dendritic cells or immature dendritic cells according to GMP (Good Manufacturing Practice) guidelines which method comprises cultivating hematopoietic progenitor cells in a sterile cultivating apparatus in the presence of at least one dendritic cell differentiation/maturation factor, said cultivating apparatus comprising a closed container having an enlarged inner surface (22, 48) forming a grow area whereby the cells adhere to the surface (22, 48).

**2.** The method of claim 1, wherein the container comprises at least two fluidal communicating chambers (16; 49, 50, 52), each chamber (16; 49, 50, 52) comprising a grow area.

**3.** The method according to claim 1 or 2, wherein said chambers (16) are formed by a basin-like means (10) and a bottom surface (12) of an adjacent basin-like means (10), preferably said basin like means have a planar bottom surface (12).

**4.** The method according to claim 3, wherein said chambers (16) are formed by a stack of said basin-like means (10).

**5.** The method according to any one of claims 1 to 4, wherein said means (18, 64) for fluidal connection connect adjacent chambers (16; 50, 52).

**6.** The method according to any one of claims 1 to 5, wherein each chamber (16) comprises at least one inlet- and one outlet opening (30).

**7.** The method according to any one of claims 1 to 6, wherein the apparatus has one common main inlet opening (36, 37) so that fluid can be delivered through said main inlet opening (36, 37) into first chamber (16, 52), from said first chamber (16, 52) into the next adjacent chamber (16, 50) and so on.

**8.** The method according to claim 1, wherein the enlarged inner surface (22, 48) of the container is formed by one or more regularly or irregularly shaped structures or particles, whereby the cells adhere to the inner surface of the container and/or to the surface of said particles.

**9.** The method according to any one of claims 1 to 7, wherein
(i) said chambers are made from plastics, preferably polycarbonate, poystyrene or polyolefin, and/or
(ii) the inner surface of the container, preferably the grow area of the inner surface including the basin like means (10) and the surface of the regularly or irregularly shaped structure or particles, are coated with an adherence mediating agent, and/or
(iii) the effective grow area of the inner surface of the container is between 25 and 10000 cm², preferably 500 and 5000 cm².

**10.** The method of claim 9, wherein the adherence mediating agent is selected from an Ig coating, including an IgG coating, and a coating with antibodies to cell surface proteins, including a coating with anti-CD14 or anti-CD16 antibodies.

**11.** The method according to any one of claims 1 to 10, wherein the hematopoietic progenitor cells are pluripotent cells, dendritic cell precursors or immature dendritic cells, preferably said hematopoietic progenitor cells are dendritic cell precursors which
(i) are obtained from CD14⁺ mononuclear cells (monocytes), from CD34⁺ cells, or directly from blood, and/or
(ii) are derivable from leukapheresis products, elutriation protocols, peripherally drawn blood or bone marrow.

**12.** The method according to any one of claims 1 to 11, wherein the cultivation comprises:
(i) applying peripheral blood mononuclear cells into the cultivation chamber and allowing them to adhere to the inner surface of the chamber,
(ii) washing the chamber in order to remove non-adhering cells,
(iii) cultivating the adhering cells in a culture medium comprising said at least one dendritic cell differentiation/maturation factors, and optionally
(iv) adding further differentiation/maturation factor(s) to the culture medium, and/or
(v) removing the culture medium, washing the adhering cells and cultivating the adhering cells in a culture medium comprising same or different dendritic cell maturation factor(s) as compared to those of step (iii).

**13.** The method according to any one of claims 1 to 12, wherein the cultivation comprises
(i) loading 3x10⁵ to 4x10⁶ progenitor cells per cm² grow area, and/or
(ii) adding 0,01 to 3 ml, preferably 0,1 to 0,5 ml, culture medium per cm² grow area so that the grow area is sufficiently covered with culture medium, preferably from 1 to 5 mm.

**15.** The method according to any one of claims 1 to 13, wherein said at least one dendritic cell maturation factor is selected from the group consisting of IL-1β, IL-6, TNF-α, PGE₂, IFN-α, lipopolysaccharides and other bacterial cell products (such as MPL (monophosphoryl lipid A) and lipoteichoic acid), phosphoryl choline, calcium ionophores, phorbol ester (such as PMA) heat-shock proteins, nucleotides (such as ATP), lipopeptides, artificial ligands for Toll-like receptors, double-stranded RNA (such as polyI:C), immunostimulatory DNA sequences, CD40 ligand, etc., and preferably is a mixture of IL-1β, IL-6, TNF-α, and PGE₂.

**15.** An apparatus for use in producing mature and stable dendritic cells according to anyone of the claims 1 to 14, comprising a closed container having an enlarged inner surface (22, 48) forming a grow area whereby the cells adhere to the surface (22, 48).

**17.** Apparatus according to claim 15, **characterized in that** the container comprises at least two fluidal communicating chambers (16; 48, 50, 52), each chamber (16; 48, 50, 52) comprising a grow area.

**18.** Apparatus according to claim 16, **characterized in that** said grow areas have a flat surface (22, 48).

**19.** Apparatus according to claim 16 or 17, **characterized in that** communicating means (18, 64, 88) are arranged between said chambers (16; 48, 50, 52) so that fluid will be equally distributed between said chambers (16; 48, 50, 52) if the container is arranged in a communicating position.

**20.** Apparatus according to any one of the claims 16 to 18, **characterized in that** the communicating means (18, 64, 88) are arranged between the chambers (16; 48, 50, 52) so that fluid will be held within that chambers (16; 48, 50, 52) if the container is arranged in a cultivating position.

**21.** Apparatus according to any one of the claims 15 to 19, **characterized in that** said chambers (16) are formed by basin-like means (10) and a bottom surface (12) of an adjacent basin-like means (10).

**22.** Apparatus according to claim 20, **characterized in that** said chambers (16) are formed by a stack of said basin-like means (10).

**23.** Apparatus according to any one of the claims 15 to 21, **characterized in that** said means for fluidal connection (18, 64) connect adjacent chambers (16; 48, 50, 52).

**24.** Apparatus according to any one of the claims 15 to 22, **characterized in that** each chamber (16) comprises at least one inlet- and one outlet opening (20).

**25.** The apparatus according to any one of the claims 15 to 23, **characterized in that** the container has one common main inlet opening (36, 76) so that fluid can be delivered through said main inlet opening (36, 76) into a first chamber (16, 52) from said first chamber (16, 52) into the next adjacent chamber (16, 50) and so on.

**26.** The apparatus according to any one of the claims 20 to 29, **characterized in that** said main inlet opening (76) is sealed by a cover (80) having a hydrophobic filter (82).

**27.** The apparatus according to claim 20, **characterized in that** the enlarged inner surface (22, 48) of the container is formed by one or more regularly or irregularly shaped structures or particles.

**28.** The apparatus according to claims 15 to 27, **characterized in that** the chambers (92) have a gas-permeable and liquid-permeable surface, preferably said chambers (92) are adjacent to gas chambers (102).

**29.** A method for preparing peripheral blood mononuclear cells, which are suitable for cultivation of dendritic cells, from leukapheresis products, said method comprising lysis of the erythrocytes within the leukapheresis product by the addition of water or aqueous ammonium chloride solution.

**30.** The method of claim 29, wherein the lysis is performed with water and
(a)the lysis is performed at 20 to 30 °C for 5 to 30 s; and/or
(b) the volume ratio leukapheresis product to water is 20:1 to 2:1, or the lysis is performed with aqueous ammonium chloride and
(a)the concentration of the ammonium chloride in the added solution is 0,5 to 5 % (w/w) preferably 1 to 3 %(w/w); and/or
(b)the lysis is performed at 25 to 50 °C for 5 to 20 min; and/or
(c) the volume ratio leukapheresis product to ammonium chloride is 20:1 to 2:1.

**31.** The method of claim 29 or 30, which further comprises quenching, and/or washing, and/or suspending the lysis product in a culture medium or aqueous salt solution.
